# EUROPEAN PATENT APPLICATION

(11) **EP 4 215 103 A1**
(43) Date of publication of application: **26.07.2023**
(21) Application number: 22305066.7
(22) Date of filing: 21.01.2022
(51) Int. Cl.: A61B 3/10

(54) **IMAGING APPARATUS AND METHOD FOR IN VIVO FULL-FIELD INTERFERENCE IMAGING OF A SAMPLE**

(71) Applicant: Sharpeye, 94250 Gentilly (FR)
(72) Inventor: MAZLIN, Viacheslav, 75013 France (FR); BOCCARA, Albert Claude, 75006 Paris (FR); BEN AISSA, Ahmed, 75009 Paris (FR); GRIEVE, Katharine Frances, 94250 Gentilly (FR); PAQUES, Michel, 75001 Paris (FR); SAHEL, José-Alain, 75013 Paris (FR); FINK, Mathias Alexandre, 92190 Meudon (FR)
(74) Representative: Icosa

(57) **Abstract**

The invention relates to an imaging apparatus (2) for *in vivo* full-field interference imaging of a sample, the imaging apparatus comprising:
a full-field OCT imaging system (8) including:
- a primary light source (14) configured to output a probe beam (20);
- a primary interferometer (16) comprising:
• a sample arm (26);
• a reference arm (28) comprising a reference reflection surface (30);
• a primary beam splitter (24),

- a camera (18) configured to acquire the at least one interference image provided by the primary interferometer (16) and representative of a sample arranged in the sample arm (26),
- a sample beam splitter (19) arranged in the sample arm;

a sample locating system (10) arranged outside the sample arm and having a sensing axis intersecting the sample beam splitter (19), the sample locating system being configured to output a location signal representative of a location of the sample along the sensing axis.

## Description

### FIELD OF INVENTION

The invention concerns an imaging apparatus for *in vivo* full-field interference imaging of a scattering sample.

The invention relates to the field of *in vivo* imaging, and more particularly to *in vivo* imaging of ophthalmic tissues.

### BACKGROUND OF INVENTION

Optical coherence tomography (OCT) is an interferometric technique which has become a powerful imaging modality. OCT has applications in a broad spectrum of areas, and in particular in applications of biomedical fields such as ophthalmology, dermatology, cardiovascular field, gastroenterology, and so on. Optical coherence tomography includes, for instance, full-field OCT.

For instance, document WO 2020/065068 A1 discloses an imaging apparatus for full-field OCT designed for *in vivo* imaging of ophthalmic tissues.

The imaging apparatus includes a full-field OCT (FF-OCT) system. Furthermore, the imaging technique implemented with this apparatus is based on the use of light backscattered by a sample (*i*.*e*., in the case of a biological sample, by the microscopic cell and tissue structures of said sample) when it is illuminated by a probe beam emitted by a light source of the FF-OCT system, said light source having low spatial and temporal coherence length. The sample is, typically, the eye of a subject.

A microscope objective is also provided in front of the sample to focus light originating from the light source onto said sample, specifically onto the cornea. Alternatively, in the case of retinal imaging, the microscope objective is removed, and the cornea and lens of the eye ensure focusing of the probe beam on the retina.

The FF-OCT system further comprises an interferometer, including a reference arm and a sample arm separated by a beam splitter. In this case, the sample is arranged in the sample arm of the interferometer. The presence of the interferometer makes it possible to generate, by an interference phenomenon, an interference optical signal representative of the light originating selectively from a given slice of the sample volume, and to eliminate the light originating from the rest of the sample. This results from the low temporal coherence of the light source, which allows to isolate the light backscattered by a virtual slice depth-wise in the sample.

The FF-OCT system further includes a camera that captures, over time, 2D interference images representative of the interference optical signal.

Then, processing of interference images acquired based on the optical interference signal allows to retrieve at least one FF-OCT image, said FF-OCT image being an *en-face* image of the sample, that is, an image of the sample in a plane orthogonal to an optical axis of the sample arm.

Furthermore, a spectral-domain OCT (SD-OCT) system is provided to track the position of the eye. This SD-OCT system includes a secondary light source, a secondary beam of which is coupled through the illumination arm of the FF-OCT apparatus so as to propagate along the probe beam and impinge onto the sample. Analysis of the photons backscattered by the sample in response to illumination by the secondary beam gives access, in real time, to a location of the sample. Knowledge of the position of the eye allows to compensate for eye movement (especially in the propagation direction of the probe beam), in real time.

Moreover, by scanning the secondary beam, using a scanner, cross-sections of the sample are also available.

However, such imaging apparatus is not fully satisfactory.

Indeed, in such imaging apparatus, the FF-OCT system and the SD-OCT system are strongly interrelated, as several optical elements are shared, which renders assembly and alignment of such apparatus very challenging.

Furthermore, in such known architecture, the scanner of the SD-OCT system is located far (typically 30 cm) from the microscope objective. As a result, only light propagating under small angles with respect to the propagation direction of the probe beam will enter the entrance pupil of the microscope objective. Consequently, the cross-sectional image provided by the SD-OCT system only has a few hundreds of pixels in the lateral dimension (*i*.*e*., a direction orthogonal to the propagation direction of the probe beam in the sample arm of the interferometer). This results in the SD-OCT system providing SD-OCT images of unsatisfying quality. Moreover, such SD-OCT system has a small field of view (around 1 mm).

Moreover, in such configuration, the scanner is not optically conjugated to the back focal plane of the objective. As a result, the secondary beam is not incident perpendicular to the sample surface, which leads to low-signal in the SD-OCT images, since perpendicular direction produces the highest back-reflectivity. Consequently, the signal-to-noise ratio in the cross-sections is too low to evaluate, in real-time, the health of the ocular layers.

Furthermore, in such known apparatus, achieving conjugation of the scanner to the image back focal plane of the objective is very impractical. Indeed, even though the addition of a 4F set-up, on the SD-OCT system side, could allow such conjugation, this would result, due to the extended path of the SD-OCT beam in the FF-OCT system (about 20 cm), in an unacceptably long 4F set-up (around 80 cm) which complicates any commercial exploitation.

Furthermore, since the SD-OCT system is coupled through the illumination arm of the FF-OCT apparatus, the beam splitter arranged in the interferometer of said FF-OCT apparatus causes a 75% signal loss on the SD-OCT system side, compared to a situation where the secondary beam output by the SD-OCT system directly impinges onto the sample. This is prejudicial to the computation of high-quality cross-sectional images of the sample, as well as to precise location of the moving sample.

A purpose of the invention is to provide an imaging apparatus that overcomes these problems.

### SUMMARY

To this end, the present invention is an imaging apparatus of the aforementioned type, comprising:
a full-field OCT imaging system for providing at least one interference image representative of an *en-face* image of the sample, the full-field OCT imaging system including:
   - a primary light source configured to output a probe beam;
   - a primary interferometer comprising:
      - a sample arm for receiving the sample;
      - a reference arm comprising a reference reflection surface;
      - a primary beam splitter for separating the sample arm and the reference arm,
   - a camera configured to acquire the at least one interference image, each being representative of a primary optical interference signal resulting, when the sample is arranged in the sample arm, from interferences produced, at each point of an imaging field, between:
      - a reference wave obtained by reflection of incident light waves of the probe beam on an elementary surface of the reference reflection surface conjugate to said point of the imaging field; and
      - a sample wave obtained by scattering of incident light waves of the probe beam by an elementary volume of a slice of the sample at a given depth, said elementary volume being conjugate to said point of the imaging field,
the imaging apparatus further comprising:
   - a sample beam splitter arranged in the sample arm, downstream the primary beam splitter in a direction from the primary light source to the primary beam splitter;
   - a sample locating system arranged outside the sample arm, the sample locating system having a respective sensing axis intersecting the sample beam splitter, the sample locating system being configured to output a location signal representative of a location of the sample along the sensing axis.

Indeed, such architecture provides a full-field OCT imaging system and a sample locating system that share a minimal number of components (namely, the sample beam splitter), rendering these two systems almost optically independent. This enables easier manufacturing, assembling and maintenance.

Furthermore, in such design, due to the very short distance between the sample locating system and the sample, the optical conjugation between the sample locating system and focal plane in the sample is improved leading to close to perpendicular direction of scanning angles and improvement of the signal.

Furthermore, this design allows to build a conjugation device with a short footprint between the beam scanner and the sample for the best optical performance and signal. As a result, scanning of the sample can be performed in a very precise way, thereby providing better location of the sample. Moreover, a larger field of view is achieved (about 4-5 mm).

Moreover, since the sample locating system is not coupled through the illumination arm of the full-field OCT imaging system, the aforementioned reduction of the signal on the sample locating system side is not experienced.

According to other advantageous aspects of the invention, the imaging apparatus includes one or more of the following features, taken alone or in any technically possible combination:
the sample locating system is an optical sample locating, the sensing axis of the sample locating system being an optical axis;
the sample beam splitter is a dichroic mirror;
the imaging apparatus further comprises a reference beam splitter arranged in the reference arm, between the primary beam splitter and the reference reflection surface, the reference beam splitter having the same optical properties as the sample beam splitter;
the reference beam splitter is identical to the sample beam splitter, the reference beam splitter and the sample beam splitter have similar orientation relatively to a propagation direction of light waves of the probe beam propagating through each of them;
the optical sample locating system includes an OCT imaging system configured to determine, for each interference image acquired by the full-field OCT imaging system, at least one cross-sectional image of the sample at a time of acquisition of the interference image;
the OCT imaging system includes:
   - a secondary light source configured to output a location beam;
   - a secondary interferometer comprising:
      - a secondary sample arm for receiving the sample, an optical axis of the secondary sample arm being the optical axis of the optical sample locating system;
      - a secondary reference arm comprising a secondary reflector;
      - a secondary beam splitter for separating the secondary sample arm and the secondary reference arm,
   - a detector configured to output the location signal, the location signal being representative of a secondary optical interference signal resulting, when the sample is arranged in the secondary sample arm, from interferences produced between:
      - a secondary reference wave obtained by reflection of incident light waves of the location beam on the secondary reflector; and
      - a secondary sample wave obtained by scattering of a first part of the location beam by elementary volumes of the sample located along a direction of propagation of the first part of the location beam;
the full-field OCT imaging system includes a first lens system arranged in the sample arm, downstream the primary beam splitter in a direction from the primary light source to the primary beam splitter, the OCT imaging system further including:
   - a beam scanner comprising a light deflection element for deflecting the location beam, the beam scanner being arranged in the secondary sample arm, between the sample beam splitter and the secondary beam splitter; and
   - a conjugation device including two lenses arranged in a 4F configuration, a back focal plane of the first lens system coinciding with a first focal plane of the conjugation device, and the light deflection element being arranged in a second focal plane of the conjugation device;
the imaging apparatus further comprises at least one adaptive lens and/or deformable lens and/or tunable lens arranged in the sample arm of the primary interferometer;
the imaging apparatus further comprises a processing unit configured to determine at least one *en-face* image of the sample based on each acquired interference image and the corresponding location signal;
the processing unit is further configured to perform a denoising on each *en-face* image, the denoising including providing the *en-face* image to a Noise2Noise neural network previously trained by:
   - acquiring, with the full-field OCT imaging system, at least one training set of *en-face* images, each training set including a first *en-face* image and a second *en-face* image of a same section of a training sample at distinct acquisition times;
   - for each training set, providing the first *en-face* image to the Noise2Noise model as an input and providing the second *en-face* image as a target output.

The invention also relates to an imaging method for *in vivo* full-field interference imaging of a scattering sample, the method comprising:
- arranging a sample in the sample arm of the full-field OCT imaging system as previously defined;
- acquiring at least one location signal representative of the position of the sample along the sensing axis over time;
- based on each location signal, determining a position of the sample over time;
- adjusting a position of the imaged slice of the sample based on the determined position of the sample.

According to other advantageous aspects of the invention, the imaging method includes one or more of the following features, taken alone or in any technically possible combination:
the sample is an *in vivo* eye of a subject;
the sample arm of the full-field OCT imaging system includes a first lens system configured to focus the probe beam on the cornea or on the retina of the eye;
the method further comprises a step of training a Noise2Noise neural network, including:
   - acquiring, with the full-field OCT imaging system, at least one training set of *en-face* images of a training sample, each training set including a first *en-face* image and a second *en-face* image of a same section of the training sample at distinct acquisition times;
   - for each training set, providing the first *en-face* image to the Noise2Noise model
      as an input and providing the second *en-face* image as a target output,
      the method further including:
   - determining at least one *en-face* image of the current sample based on each acquired interference image and the corresponding location signal; and
   - providing each determined *en-face* image to the trained Noise2Noise neural network for denoising.

### BRIEF DESCRIPTION OF THE DRAWINGS

The invention will be better understood with the attached figures, in which:
**Figure 1** is a schematic representation of a first embodiment of the imaging apparatus according to the invention;
**Figure 2** is a schematic representation of a 4F conjugation device of the imaging apparatus of figure 1;
**Figure 3A** and **Figure 3B** are *en-face* images of a corneal stroma and a trabecular meshwork, respectively, acquired by the imaging apparatus of figure 1, and **Figure 3C** is a cross-sectional image of a cornea acquired by the imaging apparatus of figure 1;
**Figure 4** is an example of an *en-face* image before and after a denoising step performed by a processing unit of the imaging apparatus of figure 1;
**Figure 5** is a schematic representation of a second embodiment of the imaging apparatus according to the invention;
**Figure 6** is a schematic representation of a third embodiment of the imaging apparatus according to the invention;
**Figures 7A** and **7B** are schematic representations of a fourth embodiment of the imaging apparatus according to the invention; and
**Figure 8** is a schematic representation of a fifth embodiment of the imaging apparatus according to the invention.

### DETAILED DESCRIPTION

### FIRST EMBODIMENT

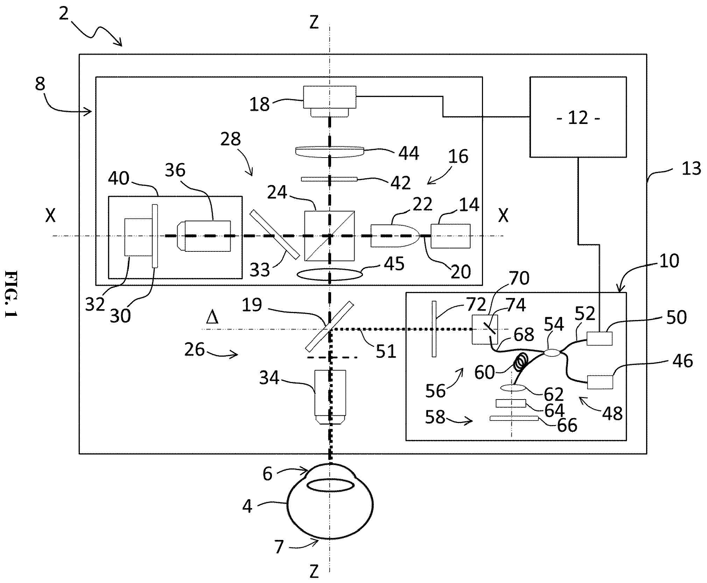

An imaging apparatus 2 according to the invention is shown on figure 1.

The imaging apparatus 2 is particularly suitable for *in vivo* imaging of a scattering sample 4, especially a moving sample, typically a subject's eye. More precisely, the imaging apparatus 2 is configured for full-field interference imaging of the sample 4.

In particular, the imaging apparatus 2 is configured to determine at least one *en-face* image (and, preferably, at least one cross-sectional image) of at least part of the sample 4, such as the cornea 6 of the *in vivo* eye 4. Preferably, the imaging apparatus 2 is also configured to provide at least one 3D image of at least part of the sample 4, for instance based on the determined *en-face* image and cross-sectional image.

The imaging apparatus 2 comprises a full-field OCT imaging system 8 (also referred to as "FF-OCT imaging system"), an optical sample locating system 10, and a processing unit 12.

The FF-OCT imaging system 8 is configured to provide at least one interference image representative of an *en-face* image of the sample 4, said *en-face* image being computed by the processing unit 12.

According to the invention, an *en-face* image of the sample 4 is an image of an in-depth section of the sample 4. Such in-depth section corresponds to a section of the sample 4 according to a plane that is orthogonal to an optical axis Z-Z of the FF-OCT imaging system 8.

Furthermore, the optical sample locating system 10 is configured to output a location signal representative of a location of the sample 4 along the optical axis Z-Z. The location signal is, for instance, an optical spectrum, or a temporal signal having an evolution that is representative of the movements of the sample.

Preferably, the processing unit 12 is also configured to compute at least one cross-sectional image of at least part of the sample 4 based on the location signal.

Preferably, the processing unit 12 is also configured to compute the at least one 3D image of at least part of the sample 4 based on a time sequence of *en-face* images and associated cross-sectional images.

Preferably, the imaging apparatus 2 further comprises a main movable platform 13 for mounting the FF-OCT imaging system 8 and the optical sample locating system 10 (for instance a motorized platform, or a manual platform that is hand-controlled by an operator), the main movable platform being movable along three independent (for instance perpendicular) directions so as to jointly translate the FF-OCT imaging system 8 and the optical sample locating system 10.

### FF-OCT imaging system 8

A description of the FF-OCT imaging system 8 will now be provided.

The FF-OCT imaging system 8 comprises a primary light source 14, a primary interferometer 16, a camera 18 and a sample beam splitter 19.

### Primary light source 14

The primary light source 14 is configured to output a probe beam 20 (dashed line on the figure), and is arranged at an input of the primary interferometer 16.

Preferably, the spectrum of the probe beam 20 has a central peak at a wavelength belonging to the [0.4 µm; 1 µm] interval, even more preferably the [0.75 µm; 1 µm] interval for eye examination.

The primary light source 14 is a source having low spatial coherence (around half the wavelength, for instance lower than 1.5 µm when using a second lens system 36 having a 0.3 numerical aperture) and a low temporal coherence length (for instance less than 20 µm). For instance, the primary light source 14 is a thermal light source (such as a halogen lamp), a light-emitting diode, or a laser followed by mode-mixing multimode fiber.

Preferably, an optical element 22 is arranged at the output of the primary light source 14 to achieve Köhler illumination.

The primary light source 14 is arranged so that the probe beam 20 emitted by said primary light source 14 reaches a primary beam-splitter 24 (described below) of the primary interferometer 16, thereby having a first part thereof propagating in a sample arm 26 of the primary interferometer 16, and a second part thereof (distinct from the first part of the probe beam 20) propagating in a reference arm 28 of the primary interferometer 16.

### Primary interferometer 16

The primary interferometer 16 includes the aforementioned primary beam splitter 24, sample arm 26 and reference arm 28. The sample arm 26 is intended to receive the sample 4.

The primary interferometer 16 is configured to generate a primary optical interference signal resulting, when the sample 4 is arranged in the sample arm 26, from interferences produced, at each point of an imaging field of the FF-OCT imaging system 8, between a sample wave obtained from the sample arm 26, and a reference wave received from the reference arm 28. The sample wave and the reference wave will be further described below.

Preferably, as shown on figure 1, the primary interferometer 16 is a Linnik interferometer. Nevertheless, the primary interferometer 16 may be any other suitable type of interferometer, such as a Michelson interferometer.

Each element of the primary interferometer 16 will now be described in more detail.

### Primary beam splitter 24

The primary beam splitter 24, which separates the sample arm 26 and the reference arm 28, is, for instance, a non-polarizing splitter cube.

### Sample arm 26

As previously stated, the sample arm 26 is intended to receive the sample 4. The sample arm has a respective optical axis, which defines the optical axis Z-Z of the FF-OCT imaging system 8.

The sample arm 26 is arranged so that the sample 4 receives, from the primary beam splitter 24, the aforementioned first part of the probe beam 20.

Furthermore, the aforementioned sample wave is obtained by scattering of incident light waves of said first part of probe beam 20 by the sample 4. More precisely, at each point of the imaging field, the corresponding sample wave is obtained by scattering (*e*.*g*., backscattering) of incident light waves of said first part of the probe beam 20 by an elementary volume of a slice of the sample 4, said elementary volume being conjugate to said point of the imaging field, all elementary volumes having the same given depth, *i*.*e*., the same coordinate along the optical axis Z-Z.

### Reference arm 28

The reference arm 28 has an optical axis X-X, and comprises a reference reflection surface 30.

The reference arm 28 is arranged so that reference reflection surface 30 receives, from the primary beam splitter 24, the aforementioned second part of the probe beam 20, distinct from the first part.

In this case, the aforementioned reference wave is obtained by reflection of incident light waves of said second part of probe beam 20 on the reference reflection surface 30. More precisely, at each point of the imaging field, the corresponding reference wave is obtained by reflection of incident light waves of said second part of the probe beam 20 on an elementary surface of the reference reflection surface 30 conjugate to said point of the imaging field.

Preferably, the reference reflection surface 30 is flat. For instance, the reference reflection surface 30 is a metallic mirror, a neutral density filter glass, or a glass plate.

Alternatively, the reference reflection surface 30 is curved, preferably with a curvature that matches the convex curvature of the cornea. This feature is advantageous, as it results in an *en-face* image that does not lie in a plane orthogonal to the Z-Z axis, but rather represents a whole cell layer of the cornea at once.

Advantageously, the reference reflection surface 30 is mounted on a piezoelectric stage 32. Such piezoelectric stage 32 may be used for phase modulation, as will later be described in relation to the processing unit 12. Preferably, the piezoelectric stage 32 is configured to operate in the [100 Hz; 10 kHz] range.

### Camera 18

The camera 18 is arranged at an output of the primary interferometer 16.

The camera 18 is configured to acquire at least one interference image representative of the aforementioned primary optical interference signal resulting, when the sample 4 is arranged in the sample arm 26, from interferences in the imaging field between the reference waves and the sample waves.

Each interference image is a 2D image which, once processed by the processing unit 12, allows to retrieve an *en-face* image of the sample 4, that is, an image of the sample in a plane orthogonal to the optical axis Z-Z of the sample arm 28 and conjugate to the imaging field.

Preferably, the camera 18 is configured to acquire the interference images at a high rate, for example with a frequency comprised between 100 Hz and 1 kHz, or even higher, depending on the dynamics of the sample 4, and more specifically the dynamics of the movements within the sample 4.

For instance, the camera 18 is a charge-coupled device (CCD) camera or a complementarity metal oxide semiconductor (CMOS) camera.

### Sample beam splitter 19

The sample beam splitter 19 is arranged in the sample arm 26 and crosses the optical axis Z-Z of the sample arm 26. More precisely, the sample beam splitter 19 is arranged downstream the primary beam splitter 24 in a direction from the primary light source 14 to the primary beam splitter 24. In other words, as shown on figure 1, when the sample 4 is arranged in the sample arm 26, the sample beam splitter 19 is located between the primary beam splitter 24 and the sample 4.

The sample beam splitter 19 is, preferably, configured to transmit light having a wavelength within a predetermined range around the central wavelength of the primary light source 14, and to reflect light having a wavelength within a predetermined range around a central wavelength of a secondary light source 46 (described below) of the optical sample locating system 10. For instance, the sample beam splitter 19 is a dichroic mirror.

### Additional optional optical elements

### Reference beam splitter 33

Advantageously, the primary interferometer 16 also comprises a reference beam splitter 33 arranged in the reference arm 28, between the primary beam splitter 24 and the reference reflection surface 30. This is advantageous, as such reference beam splitter 33 introduces, in the reference arm 28, dispersion similar to that cause by the sample beam splitter 19 in the sample arm 26, thereby improving the ability to extract *en-face* images from the interference images acquired thanks to the FF-OCT imaging system 8.

The reference beam splitter 33 has the same optical properties as the sample beam splitter 19. For instance, the reference beam splitter 33 is identical to the sample beam splitter 19.

Preferably, the reference beam splitter 33 and the sample beam splitter 19 are oriented so that they define a same angle with a propagation direction of light waves of the probe beam 20 propagating through each of them. In other words, the reference beam splitter 33 and the sample beam splitter 19 are oriented so that an angle between the plane into which the sample beam splitter 19 extends and a propagation direction of the first part of the probe beam 20 is equal to an angle between the plane into which the reference beam splitter 33 extends and a propagation direction of the second part of the probe beam 20.

More precisely, for each point of an illumination field of the probe beam 20:
- on the one hand, the angle between the propagation direction of the first part of the probe beam 20 and the plane into which lies an elementary surface of the sample beam splitter 19 onto which light originating from said point of the illumination field impinges; and
- on the other hand, the angle between the propagation direction of the second part of the probe beam 20 and the plane into which lies an elementary surface of the reference beam splitter 33 onto which light originating from said point of the illumination field impinges,
- are substantially equal.

This feature is advantageous, since the FF-OCT imaging system 8 may be sensitive to small mismatches between the sample arm 26 and the reference arm 28 (contrary to conventional SD-OCT systems). Indeed, by having the angles between the identical beam splitters 19, 33 in the sample arm 26 and the reference arm 28 match, the dispersion of the arms 26, 28 of the primary interferometer 16 are balanced. As a result, with appropriate tuning, the optical path length difference between the sample arm 26 and the reference 28 becomes smaller than the coherence length of the primary light source 14 (for instance, 8 µm), thereby allowing the FF-OCT imaging system 8 to acquire reliable interference images.

This feature also allows to provide lateral (*i*.*e*., perpendicular to the propagation of light) optical matching in the sample arm 26 and the reference arm 28, thereby further increasing the ability of the FF-OCT imaging system 8 to acquire reliable interference images.

### First and second lens systems 34, 36

Preferably, the primary interferometer 16 further comprises a first lens system 34 and a second lens system 36, arranged respectively on each of the sample arm 26 and the reference arm 28.

More precisely, the first lens system 34 is arranged so that the focal plane of said first lens system 34 matches the section of the sample 4 that is imaged, when the sample 4 is positioned in the sample arm 26. Furthermore, the first lens system 34 is arranged so that a corresponding optical axis coincides with the optical axis Z-Z of the sample arm 26.

In practice, the depth of focus of the first lens system 34 is always more extended, along the optical axis Z-Z, than the slice width which actually causes interference, due to the low temporal coherence of the primary light source 14. In other words, the camera 18 captures light from a relatively thick region of the sample 4 but optical interference signals resulting in the *en-face* image are located in a thinner sub-region of the depth of focus, the thickness of which is determined by the primary light source 14. By acquiring several camera frames with different phases, one can extract only the thin interference fringe section.

Preferably, the first lens system 34 is arranged downstream the sample beam splitter 19 in a direction from the primary beam splitter 24 to the sample beam splitter 19.

Furthermore, the second lens system 36 is arranged so that the reference reflection surface 30 is located at the focal plane of the second lens system 36.

Moreover, the second lens system 36 is arranged so that a corresponding optical axis coincides with the optical axis X-X of the reference arm 28.

Advantageously, each lens system 34, 36 has a high numerical aperture (typically around 0.3) to ensure cellular resolution while providing a large field-of-view (typically around 1 mm). For instance, each of the first lens system 34 and the second lens system 36 is a microscope objective.

Advantageously, the reference reflection surface 30 and the second lens system 36 are mounted on a motorized platform 40, movable along the optical axis X-X of the reference arm 28.

This is advantageous, as displacement of the motorized platform 40 along the optical axis X-X allows to position the imaged slice based on the position of the sample. More precisely, when imaging retina of the eye 4, displacement of the motorized platform 40 along the optical axis X-X allows to select the depth of the imaged slice (*i*.*e*., the plane orthogonal to the optical axis Z-Z that is imaged by the FF-OCT imaging system 8). Furthermore, when imaging cornea, displacement of the motorized platform 40 allows to compensate for defocus caused, in the eye, by Snell's law, and which depends on the position of the eye relative to the FF-OCT imaging system 8, so that the imaged slice falls in the current focus plane.

### Primary spectral filter 42

Preferably, the primary interferometer 16 further comprises a primary spectral filter 42 arranged between an output of said primary interferometer 16 and the camera 18.

The primary spectral filter 42 is configured to transmit light having a wavelength within a predetermined range around the central wavelength of the primary light source 14, and to block light having a wavelength outside said predetermined range, such as light emitted by the optical sample locating system 10. This prevents the camera 18 from detecting photons originating from the locating system 10.

For instance, the primary spectral filter 42 is an optical bandpass filter.

### Third lens system 44

Preferably, the primary interferometer 16 also includes a third lens system 44, such as an achromatic doublet, arranged between an output of said primary interferometer 16 and the camera 18.

The third lens system 44 has a focal length selected to conjugate a detecting surface of the camera 18 with the planes situated at the foci of the first and second optical systems 34, 36. For instance, the focal length of the third lens system 44 is a few hundreds of millimeters, typically 300 mm.

Such third lens system 44 (typically a tube lens) also allows to modify the magnification and field-of-view that the camera sees 18, which is advantageous. Indeed, the first and second lens systems 34, 36 are generally "infinity corrected". For instance, the system of Nikon microscope objective lens 34 (having the Tube Lens Focal Length equal to 200 mm) together with the 200 mm tube lens 44 would produce 10X magnified view, while the system with the 300 mm tube lens 44 would give a 15X magnification.

### Tunable lens 45

Preferably, the primary interferometer 16 further includes a tunable (i.e., adaptive) lens 45 arranged in the sample arm 26.

The tunable lens 45 is operable by the processing unit 12, advantageously in combination with the motorized platform 40, to compensate for defocus on the FF-OCT imaging system side, due to the fact that the eye does not have the same refractive index as air. More precisely, the tunable lens 45 is operable to control the position of the focus point of the first part of the probe beam 20, which impinges on the sample 4.

Optionally, the tunable lens 45 may be configured to provide similar functionalities as those of the third lens system 44. In this case, the third lens system 44 may be removed.

### Optical sample locating system 10

A description of the locating system 10 will now be provided.

As stated previously, the optical sample locating system 10 (also referred to as "locating system") is configured to output a location signal representative of a location of the sample 4 along the optical axis Z-Z.

The locating system 10 is arranged outside the sample arm 26, and has a respective optical axis Δ intersecting the sample beam splitter 19. Due to the presence of the sample beam splitter 19, a location beam 51 emitted by the locating system 10 along its optical axis Δ is reflected by the sample beam splitter 19 so as to propagate substantially parallel to the optical axis Z-Z of the FF-OCT imaging system 8. Therefore, after reflection on the sample beam splitter 19, the optical axis Δ and the optical axis Z-Z are co-located.

It is to be noted that the direction of propagation of the location beam 51 (i.e., the optical axis Δ) is truly parallel to the optical axis Z-Z only for a single orientation of a deflection element 74 (described below) of the locating system 10. For all the other positions of the deflection element 74 (all of them being used during acquisition of the location signals leading to a 2D cross-sectional image of the sample 4), there exists an angle between the optical axis Δ and the optical axis Z-Z.

Conversely, light from the sample 4 (with appropriate wavelength) is able to reach the locating system 10 after being reflected by the sample beam splitter 19.

Preferably, the locating system 10 includes an OCT imaging system (such as a spectral-domain OCT system (also referred to as "SD-OCT system"), a swept-source OCT system or a time-domain OCT system), as will be described below.

In this case, the locating system 10 includes the aforementioned secondary light source 46, a secondary interferometer 48 and a detector 50.

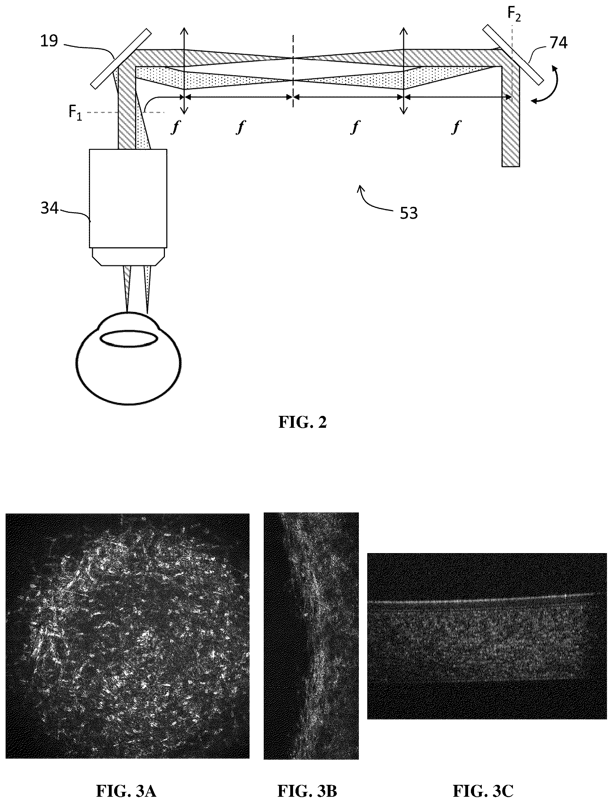

Preferably, the locating system 10 further includes a conjugation device 53, shown on figure 2.

### Secondary light source 46

The secondary light source 46 is configured to output the aforementioned location beam 51 (dotted line on the figure), and is arranged at an input of the secondary interferometer 48.

Advantageously, the location beam has a spectrum that at least partially does not overlap with a spectrum of the light emitted by the primary light source 14.

For instance, the spectrum of the location beam 51 has a central peak at a wavelength belonging to the [870 nm; 1000 nm] interval while the primary light source 14 emits light in the [750 nm; 860 nm] interval.

Preferably, the secondary light source 46 is a spatially coherent light source. For instance, the secondary light source 46 is a super-luminescent diode or a swept laser source (when using a swept-source OCT system).

Preferably, in the case where the locating system 10 includes an SD-OCT system, the secondary light source 46 has low temporal coherence (*i*.*e*., a broad spectrum compared to its central wavelength). Furthermore, in the case of a swept laser source, at each moment, the location beam has quite large temporal coherence, but is swept over a broad spectrum.

The secondary light source 46 is coupled to an input of the secondary interferometer 48, for instance through a first optical fiber 52.

The secondary light source 46 is arranged so that the location beam 51 emitted by secondary light source 46 reaches a secondary beam-splitter 54 (described below) of the secondary interferometer 48, thereby having a first part thereof propagating in a secondary sample arm 56 of the secondary interferometer 48, and a second part thereof (distinct from the first part of the location beam 51) propagating in a secondary reference arm 58 of the secondary interferometer 48.

### Secondary interferometer 48

The secondary interferometer 48 includes the aforementioned secondary beam splitter 54, secondary sample arm 56 and secondary reference arm 58.

The secondary beam splitter 54 separates the secondary sample arm 56 and the secondary reference arm 58. Moreover, the secondary sample arm 56 is intended to receive the sample 4.

The secondary interferometer 48 is configured to produce a secondary optical interference signal resulting, when the sample 4 is arranged in the secondary sample arm 56, from interferences produced between a secondary sample wave obtained from the secondary sample arm 56, and a secondary reference wave received from the secondary reference arm 58. The secondary sample wave and the secondary reference wave will be further described below.

Due to the architecture of the imaging apparatus 2, arranging the sample 4 in the sample arm 26 of the primary interferometer 16 is equivalent to arranging the sample 4 in the secondary sample arm 56 of the secondary interferometer 48.

### Secondary reference arm 58

Preferably, the secondary reference arm 58 includes an optical fiber 60, an optional lens 62, an optional dispersion compensation plate 64 and a secondary reflector 66 (such as a metalized mirror) arranged in series.

In this case, the aforementioned secondary reference wave is obtained by reflection of incident light waves of the second part of the location beam on the secondary reflector 66.

Advantageously, the length of the optical fiber 60 is chosen so that an optical path between the secondary beam splitter 54 and the secondary reflector 66 is equal to an optical path between the secondary beam splitter 54 and the cell layer in the sharp focus of first lens system 34, that is the cell layer that is currently imaged. Preferably, the length of the optical fiber 60 is chosen so that the optical path between the secondary beam splitter 54 and the secondary reflector 66 is equal to an optical path between the secondary beam splitter 54 and a predetermined position where the sample 4 is supposed to be located during operation of the imaging apparatus 2.

### Secondary sample arm 56

Preferably, the secondary sample arm 56 includes an optical fiber 68, a beam scanner 70 and a secondary spectral filter 72 arranged in series.

In this case, the aforementioned secondary sample wave is obtained by scattering of incident light waves of the first part of the location beam by the sample 4. More precisely, the sample wave is obtained by scattering of incident light waves of the first part of the location beam by elementary volumes of the sample 4 located along the direction of propagation of the first part of the location beam after deflection by the beam scanner 70.

The beam scanner 70 is arranged between the sample beam splitter 19 and the secondary beam splitter 54. The beam scanner 70 is configured to scan the location beam in a plane orthogonal to the optical axis X-X. More precisely, the beam scanner 70 includes a deflection element 74 (typically, a mirror) for deflecting the location beam 51. Consequently, due to the reflection of the location beam on the sample beam splitter 19, operation of the beam scanner 70 (*i*.*e*., movement of the deflection element 74) results, at the sample 4, in a scanning of the location beam in a plane orthogonal to the optical axis Z-Z, more precisely in a scanning of the location beam along multiple lines in a plane orthogonal to the optical axis Z-Z, each line corresponding to the direction of propagation of the first part of the location beam 51.

For instance, the beam scanner 70 is configured to scan a line every 10 µs approximately. In other words, the beam scanner 70 is configured to scan the eye 4 during a time interval that is shorter than a fluctuation time constant of the eye position, that is to say shorter than the typical time during which the eye position can fluctuate with a large range (greater than 10 micrometers), typically around 10 ms.

The secondary spectral filter 72 is configured to transmit light having a wavelength within a predetermined range around the central wavelength of the secondary light source 46, and to block light having a wavelength outside said predetermined range, such as light emitted by the FF-OCT imaging system 8. For instance, the secondary spectral filter 72 is an optical bandpass filter. This prevents the detector 50 from detecting photons originating from the FF-OCT imaging system 8.

### Detector 50

The detector 50 is coupled to an output of the secondary interferometer 48.

The detector 50 is configured to detect the secondary optical interference signal and to output the corresponding location signal over time.

For instance, in the case where the locating system 10 is a time-domain OCT system of a swept-source OCT system, the detector 50 is a photodetector such as a photodiode. Alternatively, in the case where the locating system 10 isa spectral-domain OCT system, the detector 50 is a spectrometer.

### Conjugation device 53

The conjugation device 53 includes two lenses arranged in a 4F configuration, as shown on figure 2.

More precisely, the conjugation device 53 is arranged so that a back focal plane of the first lens system 34 coincides with a first focal plane F₁ of the conjugation device 53, and the deflection element 74 of the beam scanner 70 is arranged in a second focal plane F₂ of the conjugation device 53.

Thanks to the presence of the conjugation device 56, the location beam 51 is scanned, by the beam scanner 70, in a plane orthogonal to the optical axis X-X and located at about the same depth as the imaging field determined by the first lens system 34. This is shown by the crosshatched beam and the dotted beam on figure 3, which correspond to the path of the location beam 51 at two different deflection angles of the deflection element 74. As a result, the deflection element 74 is virtually located at the back focal plane of the first lens system 34. Consequently, even if the beam scanner 70 is located far from said back focal plane, a problem where only light propagating under small angles with respect to the propagation direction of the probe beam will enter the entrance pupil of the first lens system 34 is solved. Furthermore, lenses having short focal length (about 3 cm) can be used, thereby resulting in a very compact conjugation device 53.

### Processing unit 12

According to the present invention, the expression "processing unit" should not be construed to be restricted to hardware capable of executing software, and refers in a general way to a processing device, which can for example include a microprocessor, an integrated circuit, or a programmable logic device (PLD). The processing unit may also encompass one or more Graphics Processing Units (GPU) or Tensor Processing Units (TSU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

The processing unit 12 is configured to compute each *en-face* image based on each interference image output by the FF-OCT imaging system 8.

To do so, the processing unit is configured to control the piezoelectric stage 32 so as to displace the reference reflection surface 30 along the optical axis X-X, thereby modulating the reference wave. The amplitude of such modulation is set according to a predetermined phase demodulation scheme, for instance 2-phase demodulation scheme or 4-phase demodulation scheme.

For instance, in a 2-phase scheme, the processing unit 12 is configured to determine the *en-face* image from the two interference images acquired by the camera 18: the first interference image is captured with the reference reflection surface 30 is in a first position, and the second interference image is captured with the reference reflection surface 30 shifted by an optical phase of π, *i*.*e*., half the central wavelength of the probe beam 20.

As another example, in 4-phase scheme, the processing unit 12 is configured to determine the *en-face* image from the four interference images acquired by the camera 18, the images being captured sequentially. If one assumes that the phase of the initial frame is 0, then the processing unit 12 is configured to control the piezoelectric stage 32 and the camera 18 so that the phase shifts for the next frames, relatively to the first frame, are π/2, π and 3 π/2.

The processing unit 12 is further configured to compute a reflectivity of the sample structures within the coherence volume (*i.e*., the plane of interest) based on a result of subtracting said phase shifted images by pairs. The resulting *en-face* image, an example of which is shown on figure 3A, is a representation of the value of the computed reflectivity across the plane of interest. More precisely, figure 3A shows a healthy corneal stroma of a human eye.

Moreover, figure 3B shows a trabecular meshwork, which is the area at the limbus of the eye that is located at the area merging the corneal endothelium and iris.

Figure 3B proves that the imaging apparatus 2 is able to provide images at a quasi-cell-resolution of this difficult to precisely locate small area (about 0.5 mm in length). This is achieved thanks to the ability of the imaging apparatus to precisely track the position of the eye 4, thanks to the optimized use of the locating system 10.

Imaging trabecular meshwork is key for diagnosing glaucoma, a condition that is detrimental to vision and that is extremely difficult to diagnose at an early stage. The ability to image the trabecular meshwork at high resolution (which is not provided by known imaging systems) allows to detect glaucoma at an early stage and avoid the risks of losing vision.

Alternatively, or in addition, in the case of a sample moving along the Z-Z axis (typically, the eye exhibiting the natural physiological eye movements), the processing unit 12 is configured to compute each *en-face* image based on each interference image output by the FF-OCT imaging system 8, without modulation of the position of the reference reflection surface 30. In this case, small movements of the sample along the Z-Z axis result in interference images that are phase-shifted with respect to each other, thereby allowing computation of the *en-face* images.

Preferably, the processing unit 12 is also configured to control the position of the motorized platform 40, based on the location signal, to compensate for sample movement, so that a same sample slice is imaged over time.

More precisely, for a given position of the deflection element 74, the detector 50 acquires a location signal representative of a line corresponding to the direction of propagation of the first part of the location beam 51. The processing unit 12 is classically configured to reconstruct the portion of the sample that is intersected by said line.

Preferably, the processing unit 12 further controls the beam scanner 70 so that the location signal for various positions of the deflection element 74 (that is, various neighboring lines) is acquired. The processing unit 12 is further configured to compute, based on said plurality of location signals, at least one cross-sectional image of at least part of the sample 4. An example of such cross-sectional image of the cornea is shown on figure 3C.

Furthermore, the processing unit 12 is configured to segment the cross-sectional image so as to identify a position of at least one given structure (such as a cellular structure) of the sample 4. As a result, by comparing each segmented cross-sectional images to a predetermined target central position, the processing unit 12 is able to determine, over time, the position, along the optical axis Z-Z, the position of each segmented structure.

Preferably, the processing unit 12 is further configured to perform image processing on each computed *en-face* image to correct noise arising from the operation of the FF-OCT imaging system 8.

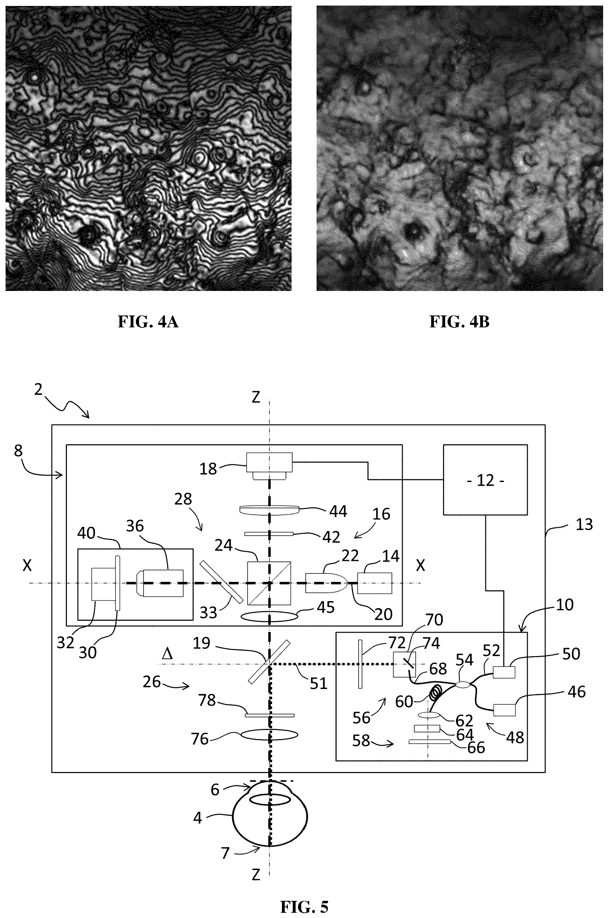

Indeed, as shown on figure 4A, the *en-face* image is generally noisy. Such noise mainly includes Gaussian noise and interference fringes noise and background.

The Gaussian noise originates from the detection, that is from the camera 18. This type of noise, which is not specific to FF-OCT, is present in any images taken by a camera. Gaussian noise is uncorrelated, which means that noise in one pixel is independent from noise in any other pixel.

Interference fringe noise, which can be seen, on figure 4A, as stripes of alternating low and high intensity, results from the fact that, due to the movements of the sample 4 (like the eye), random phase fluctuations are introduced in the interference images acquired by the camera 18, in addition to those caused by the piezoelectric stage 132. These fluctuations result in a random location and frequency, over time, of the interference fringes. Furthermore, since these phase fluctuations are random, they cannot be simply removed, for instance through the use of the 4-phase demodulation scheme, so that residual interference fringes remain.

Interference fringes are correlated: for example, if one pixel is very bright, there is high chance that the nearby pixels are bright as well.

In order to perform noise correction, the processing unit 12 is configured to implement an artificial intelligence model, such as Noise2Noise deep learning model.

More precisely, the model has been previously trained using a set of training images, for instance *in vivo* human corneal FF-OCT images (such as stroma and endothelium).

More precisely, using the full-field OCT imaging system 10, at least one training set of *en-face* images of a training sample is acquired. Each training set includes a first *en-face* image and a second *en-face* image of a same section of the training sample 4 at distinct acquisition times. Due to the high speed of the camera 18 (around 500 frames/second), said *en-face* images can be regarded as if the eye movements were frozen (*i.e*., in the same location) for a few frames. In this case, for each training set, the first *en-face* image and the second *en-face* image exhibit different noises: this is due, on the one hand, to Gaussian noise (which is different from a camera frame to the other), and, on the other hand, to interference noise (which is caused by imperfect demodulation scheme and/or sub-micron movements of the eye from frame to frame leading to phase fluctuations from an acquired interference image to the other).

Then, to train the Noise2Noise model, for each training set, the first *en-face* image is provided as an input, and the second *en-face* image is provided as a target output.

This is advantageous. Indeed, during such training, the Noise2Noise model tries to learn how to create one specific noisy pattern from another noisy pattern. Since noise is random, the model cannot efficiently learn this transformation, and it ends up learning to transform the noisy pattern into the average of all noisy patterns, which is a clean image. Thank to such training, both spatially uncorrelated noise (Gaussian) and correlated noise (interference fringes) are removed, as shown on figure 4B, which is the result of denoising the *en-face* image of figure 4A. This is due to the ability of the model to learn the statistics of the correlated noise, as well as the statistics of the typical structures of the sample visible in the images (*e*.*g*., eye structures).

### Operation

Operation of the imaging apparatus 2 will now be described.

During a configuration step, alignment of the FF-OCT imaging system 8 and the optical sample locating system 10 is performed.

Moreover, during the configuration step, a modulation/demodulation scheme is preferably chosen by an operator and stored into the processing unit 12.

Preferably, in the case where the processing unit 12 is intended to perform noise correction, configuration of the Noise2Noise neural network is performed during the configuration step. More precisely, the neural network is trained, as described previously.

Then, the sample 4 is arranged in the sample arm 26 of the FF-OCT imaging system 8 and the secondary sample arm 56 of the optical sample locating system 10.

Then, the processing unit 12 controls the piezoelectric stage 32 to displace the reference reflection surface 30 along the optical axis X-X, thereby modulating the reference wave. The processing unit 12 also controls the camera 18 so that it acquires interference images in accordance with the predetermined modulation scheme, that is when the reference reflection surface 30 is in positions leading to a predetermined phase shift of the reference wave.

The processing unit 12 also controls the beam scanner 70 so that the location beam 51 scans the sample 4. The processing unit 12 simultaneously controls the detector 50 so that the location signal representative of each line being currently scanned by the location beam 51 is output.

Then, the processing unit 12 computes each *en-face* image based on each interference image output by the FF-OCT imaging system 8.

The processing unit 12 also computes, based on said plurality of location signals, the cross-sectional image of the sample 4. Furthermore, the processing unit 12 segments the computed cross-sectional image so as to identify a position of at least one given structure of the sample 4.

The processing unit 12 further compares the position of the segmented cross-sectional image to a predetermined target central position to determine whether the position of each segmented structure changes over time.

If the position changes, the processing unit 12 controls the position of the motorized platform 40 to compensate for sample movement, so as to compensate for defocus.

Preferably, the processing unit 12 further implements the trained Noise2Noise neural network to perform noise correction on each computed *en-face* image. More precisely, each computed *en-face* image is provided at the input of the neural network, which outputs a denoised image.

Alternatively, as previously mentioned, modulation of the reference reflection surface 30 is not performed. In this case, the processing unit 12 relies on phase shifts introduced, from one interference image to the other, by natural movement of the sample along the optical axis Z-Z to compute the *en-face* images.

### SECOND EMBODIMENT

A second embodiment of the imaging apparatus according to the invention is shown on figure 5. Numerical references that are shared with figure 1 correspond to the same elements.

The imaging apparatus 2 of figure 5 is configured to determine at least one *en-face* image and, preferably at least one cross-sectional image, of the retina 7 of the *in vivo* eye 4.

To do so, the imaging apparatus 2 of figure 5 differs from the imaging apparatus of figure 1 in that the first lens system 34 does not comprise a microscope objective. In this case, the refractive elements of the eye focus the first part of the probe beam 20 on the retina, thereby operating as the first lens system 34.

Preferably, the imaging apparatus further includes an adaptive lens 76 (for example a liquid lens) and/or a rotating glass plate 78 inserted in the sample arm 26 to compensate for aberrations and dispersion mismatch introduced by the eye 4 with respect to the dispersion of the second lens system 36 in the reference arm 28.

If the optional conjugation device 53 is provided, then the conjugation device 53 is arranged so that the first focal plane F₁ of the conjugation device 53 coincides with the cornea-lens of the eye 4.

Furthermore, in such imaging apparatus, the processing unit 12 is advantageously configured so that, in operation, if the processing unit 12 determines that the sample 4 has moved (for instance by determining that a segmented structure of a cross-section of the sample has moved with respect to a predetermined target central position), the processing unit 12 controls the motorized platform 40 so that the plane of the retina 7 from which the interference image (of the FF-OCT imaging system 8) arises remains at the same position within the sample 4.

### THIRD EMBODIMENT

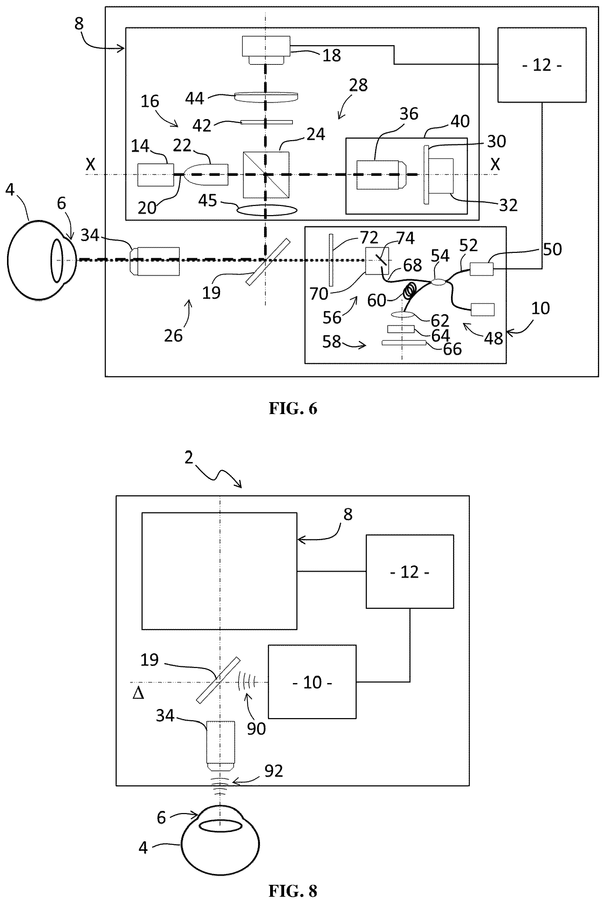

A third embodiment of the imaging apparatus according to the invention is shown on figure 6. Numerical references that are shared with figure 1 correspond to the same elements.

The imaging apparatus 2 of figure 6 only differs from the imaging apparatus of figure 1 in that the FF-OCT imaging system 8, the locating system 10 and the sample beam splitter 19 are arranged so that the probe beam 20 is reflected by the sample beam splitter 19 to impinge onto the sample 4, while the location beam 51 propagates through the sample beam splitter 19 to impinge onto the sample 4.

In this case, the reference arm 28 does not include a reference beam splitter, since the probe beam 20, in the sample arm 26, does not undergo propagation through the sample beam splitter 19 that would cause dispersion requiring compensation.

Such configuration can also be applied using the FF-OCT imaging system 8 of the imaging apparatus 2 of figure 5 that is specifically configured for retinal imaging.

### FOURTH EMBODIMENT

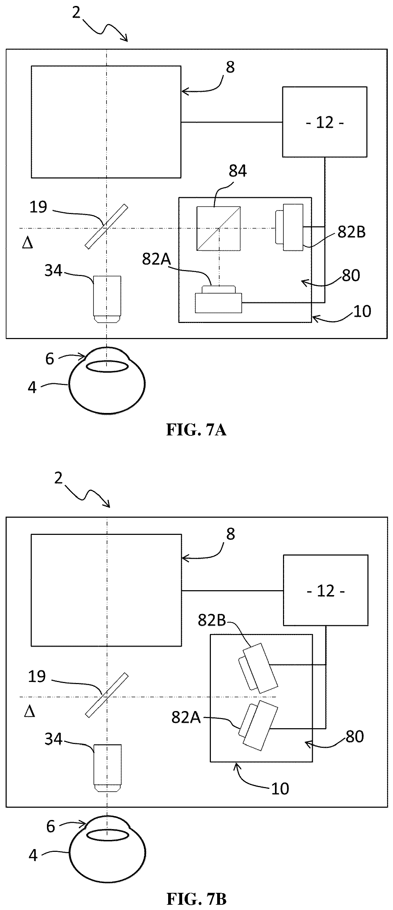

A fourth embodiment of the imaging apparatus according to the invention is shown on figure 7. Numerical references that are shared with figure 1 correspond to the same elements.

The imaging apparatus 2 of figure 7 only differs from the imaging apparatus of figure 1 in that the locating system 10 does not include an OCT imaging system, but rather a stereoscopic imager 80 having, as an optical axis, the optical axis Δ. In this case, location of the position of the sample 4 along the optical axis Z-Z is achieved thanks to the depth determination capabilities offered by stereoscopic vision.

For instance, as shown on figure 7A, the stereoscopic imager 80 comprises two cameras 82A, 82B coupled through a beam splitter 84, thereby providing stereoscopic vision.

Alternatively, as shown on figure 7B, the cameras 82A, 82B are located on either side of the optical axis Δ, thereby providing stereoscopic vision.

### FIFTH EMBODIMENT

A fifth embodiment of the imaging apparatus according to the invention is shown on figure 8. Numerical references that are shared with figure 1 correspond to the same elements

In this embodiment, the locating system 10 includes an active position sensing device configured to emit an interrogation wave 90 along the axis Δ (or "sensing axis"). The interrogation wave is reflected by the sample beam splitter 19 so that it impinges onto the sample 4.

The active position sensing is further configured to detect a response wave 92 backscattered by the sample 4 and reflected by the sample beam splitter 19 to propagate along the sensing axis Δ towards the locating system 10. Consequently, based on the detected response wave 92, the locating system 10 outputs the location signal representative of the position of the sample 4 along the Z-Z axis.

The interrogation wave may be light (*e*.*g*., laser light) or an acoustic wave.

For instance, the locating system 10 is configured to determine the location signal based on time of flight.

## Claims

1. An imaging apparatus (2) for *in vivo* full-field interference imaging of a scattering sample (4), the imaging apparatus (2) comprising:
a full-field OCT imaging system (8) for providing at least one interference image representative of an *en-face* image of the sample, the full-field OCT imaging system (8) including:
- a primary light source (14) configured to output a probe beam (20);
- a primary interferometer (16) comprising:
• a sample arm (26) for receiving the sample (4);
• a reference arm (28) comprising a reference reflection surface (30);
• a primary beam splitter (24) for separating the sample arm (26) and the reference arm (28),
- a camera (18) configured to acquire the at least one interference image, each being representative of a primary optical interference signal resulting, when the sample (4) is arranged in the sample arm (26), from interferences produced, at each point of an imaging field, between:
• a reference wave obtained by reflection of incident light waves of the probe beam (20) on an elementary surface of the reference reflection surface (30) conjugate to said point of the imaging field; and
• a sample wave obtained by scattering of incident light waves of the probe beam (20) by an elementary volume of a slice of the sample (4) at a given depth, said elementary volume being conjugate to said point of the imaging field,
the imaging apparatus (2) being **characterized in that** it further comprises:
- a sample beam splitter (19) arranged in the sample arm (26), downstream the primary beam splitter (24) in a direction from the primary light source (14) to the primary beam splitter (24);
- a sample locating system (10) arranged outside the sample arm (26), the sample locating system (10) having a respective sensing axis (Δ) intersecting the sample beam splitter (19), the sample locating system (10) being configured to output a location signal representative of a location of the sample (4) along the sensing axis (Δ).

2. The imaging apparatus (2) of claim **1**, wherein the sample locating system (10) is an optical sample locating, the sensing axis (Δ) of the sample locating system (10) being an optical axis.

3. The imaging apparatus (2) of claim **2**, wherein the sample beam splitter (19) is a dichroic mirror.

4. The imaging apparatus (2) according to claim **2** or **3**, further comprising a reference beam splitter (33) arranged in the reference arm (28), between the primary beam splitter (24) and the reference reflection surface (30), the reference beam splitter (33) having the same optical properties as the sample beam splitter (19).

5. The imaging apparatus (2) of claim **4**, wherein the reference beam splitter (33) is identical to the sample beam splitter (19), the reference beam splitter (33) and the sample beam splitter (19) have similar orientation relatively to a propagation direction of light waves of the probe beam (20) propagating through each of them.

6. The imaging apparatus (2) according to any one of claims **2** to **5**, wherein the optical sample locating system (10) includes an OCT imaging system configured to determine, for each interference image acquired by the full-field OCT imaging system (8), at least one cross-sectional image of the sample (4) at a time of acquisition of the interference image.

7. The imaging apparatus (2) of claim **6**, wherein the OCT imaging system (10) includes:
- a secondary light source (46) configured to output a location beam (51);
- a secondary interferometer (48) comprising:
• a secondary sample arm (56) for receiving the sample (4), an optical axis of the secondary sample arm (56) being the optical axis of the optical sample locating system (10);
• a secondary reference arm (58) comprising a secondary reflector (66);
• a secondary beam splitter (54) for separating the secondary sample arm (56) and the secondary reference arm (58),
- a detector (50) configured to output the location signal, the location signal being representative of a secondary optical interference signal resulting, when the sample (4) is arranged in the secondary sample arm (56), from interferences produced between:
• a secondary reference wave obtained by reflection of incident light waves of the location beam on the secondary reflector (66); and
• a secondary sample wave obtained by scattering of a first part of the location beam by elementary volumes of the sample (4) located along a direction of propagation of the first part of the location beam (51).

8. The imaging apparatus (2) according to claim **7**, wherein the full-field OCT imaging system (8) includes a first lens system (34) arranged in the sample arm (26), downstream the primary beam splitter (24) in a direction from the primary light source (14) to the primary beam splitter (24), the OCT imaging system further including:
- a beam scanner (70) comprising a light deflection element (74) for deflecting the location beam (51), the beam scanner (70) being arranged in the secondary sample arm (56), between the sample beam splitter (19) and the secondary beam splitter (54); and
- a conjugation device (53) including two lenses arranged in a 4F configuration, a back focal plane of the first lens system (34) coinciding with a first focal plane (F₁) of the conjugation device (53), and the light deflection element (74) being arranged in a second focal plane (F₂) of the conjugation device (53).

9. The imaging apparatus (2) according to any one of claims **1** to **8**, further comprising at least one adaptive lens and/or deformable lens and/or tunable lens (45) arranged in the sample arm (26) of the primary interferometer (16).

10. The imaging apparatus (2) according to any one of claims **1** to **9**, further comprising a processing unit (12) configured to determine at least one *en-face* image of the sample (4) based on each acquired interference image and the corresponding location signal.

11. The imaging apparatus (2) according to claim **10,** wherein the processing unit (12) is further configured to perform a denoising on each *en-face* image, the denoising including providing the *en-face* image to a Noise2Noise neural network previously trained by:
- acquiring, with the full-field OCT imaging system (8), at least one training set of *en-face* images, each training set including a first *en-face* image and a second *en-face* image of a same section of a training sample (4) at distinct acquisition times;
- for each training set, providing the first *en-face* image to the Noise2Noise model as an input and providing the second *en-face* image as a target output.

12. A method for *in vivo* full-field interference imaging of a scattering sample (4), the method comprising:
- arranging a sample (4) in the sample arm (26) of the full-field OCT imaging system (8) of any one of claims **1** to **11**;
- acquiring at least one location signal representative of the position of the sample (4) along the sensing axis over time;
- based on each location signal, determining a position of the sample (4) over time;
- adjusting a position of the imaged slice of the sample based on the determined position of the sample.

13. The method of claim **12**, wherein the sample (4) is an *in vivo* eye of a subject.

14. The method of claim **13**, wherein the sample arm (26) of the full-field OCT imaging system (8) includes a first lens system (34) configured to focus the probe beam (20) on the cornea (6) or on the retina (7) of the eye.

15. The method of any one of claims **12** to **14**, further comprising a step of training a Noise2Noise neural network, including:
- acquiring, with the full-field OCT imaging system (8), at least one training set of *en-face* images of a training sample, each training set including a first *en-face* image and a second *en-face* image of a same section of the training sample at distinct acquisition times;
- for each training set, providing the first *en-face* image to the Noise2Noise model as an input and providing the second *en-face* image as a target output,
the method further including:
- determining at least one *en-face* image of the current sample (4) based on each acquired interference image and the corresponding location signal; and
- providing each determined *en-face* image to the trained Noise2Noise neural network for denoising.
